# EUROPÄISCHE PATENTSCHRIFT

(11) **EP 2 254 987 B1**
(45) Veröffentlichungstag und Bekanntmachung des Hinweises auf die Patenterteilung: **10.04.2013**
(21) Anmeldenummer: 09723875.2
(22) Anmeldetag: 23.03.2009
(51) Int. Cl.: C12M 3/00

(54) **PERFUNDIERBARER BIOREAKTOR ZUR HERSTELLUNG UND/ODER KULTIVIERUNG EINES MENSCHLICHEN ODER TIERISCHEN BLUTGEFÄSSES UND/ODER EINES MENSCHLICHEN ODER TIERISCHEN GEWEBES**
PERFUSABLE BIOREACTOR FOR THE PRODUCTION AND/OR CULTIVATION OF A HUMAN OR ANIMAL BLOOD VESSEL AND/OR A HUMAN OR ANIMAL TISSUE
BIORÉACTEUR À PERFUSION POUR FABRICATION ET/OU CULTURE D'UN VAISSEAU SANGUIN HUMAIN OU ANIMAL ET/OU D'UN TISSU HUMAIN OU ANIMAL

(30) Priorität: 25.03.2008 DE 102008015633
(43) Veröffentlichungstag der Anmeldung: 01.12.2010
(73) Patentinhaber: Novatissue Gmbh, 18055 Rostock (DE)
(72) Erfinder: FRERICH, Bernard, 04277 Leipzig (DE)
(74) Vertreter: Müller, Volkmar
(86) Internationale Anmeldenummer: PCT/EP2009/002110
(87) Internationale Veröffentlichungsnummer: WO 2009/118141

(56) Entgegenhaltungen:
- EP-A1- 1 693 025
- WO-A1-97/49799
- WO-A1-03/001060
- WO-A1-2004/012782
- WO-A2-00/66036
- WO-A2-01/09282
- WO-A2-2005/040332
- WO-A2-2005/087912
- DE-A1- 19 964 113
- US-A1- 2006 258 004

## Beschreibung

Die Erfindung betrifft einen perfundierbaren Bioreaktor zur Herstellung und/oder Kultivierung menschlicher oder tierischer Gewebe oder Gewebeäquivalente, vorzugsweise in Kombination mit einem menschlichen oder tierischen Blutgefäß oder Blutgefäßäquivalent.

"Konstrukte" im Sinne der Erfindung sind artifiziell hergestellte dreidimensionale Gewebeäquivalente, die lebende Zellen in einer dreidimensionalen Matrix enthalten, insbesondere Kombinationen aus Gerüsten und lebenden Zellen, ggf. auch kombiniert mit Matrixfaktoren.
Im Sinne der Erfindung werden die Bezeichnungen Blutgefäßäquivalente und Blutgefäßwandäquivalente analog dieser Definition verwendet.

Für die in vitro Herstellung von Geweben und Blutgefäßen wurden bis heute verschiedenste Typen von perfundierbaren Bioreaktoren entwickelt. Allerdings konzentrierte man sich bisher hauptsächlich auf die Herstellung von Bioreaktoren mit starren Wandungen. Deshalb sind viele Belastungen und Einflüsse auf das in vitro wachsende Gewebe unphysiologisch. Doch es sind gerade mechanische Belastungen, welche einen nicht zu unterschätzenden Einfluss auf das Gewebewachstum in vivo und in vitro haben und ebenfalls modelliert werden sollten.
Dieses Problem spielt insbesondere eine Rolle für Weichgewebe und Blutgefäße. Die Bereitstellung oder Herstellung von Ernährung und Durchblutung durch Blutgefäße ist ein wesentliches ungelöstes Problem beim Tissue Engineering, d. h. der Zell- und Gewebezüchtung. Es besteht daher ein Bedarf an Bioreaktoren, in denen ein versorgendes Blutgefäß oder eine Blutgefäßwand in Kombination mit einem beliebigen Gewebe ("Gefäß-Gewebeeinheit") kultiviert werden kann und die zudem den physikalischen/mecbanischen Ansprüchen eines weichgewebigen und/oder vaskulären/mikrovaskutären Engineering gerecht werden. Dieser Bedarf besteht über die regenerative Medizin hinaus auch für die Testung pharmakologischer Substanzen, insbesondere solcher, bei denen die Wechselwirkungen zwischen Gefäße und Geweben eine Rolle spielen, wie z.B. für Kreislauferkrankungen oder Adipositas. Auch für die Grundlagenforschung zu solchen Erkrankungen wäre eine solches Modell wünschenswert und es wäre damit u. U. möglich, Tierversuche einzusparen.

In bisherigen Bioreaktoren sind zwar bereits pulsatorische Perfusionen realisiert worden, die den Blutdruck simulieren sollen, insbesondere um artifizielle Gefäßkonstrukte an die Blutdruckkräfte in vivo zu konditionieren. In dem Umfeld einer starren Bioreaktorwand sind diese jedoch häufig nicht physiologisch oder werden unpbysiologisch reflektiert, was auch zur Zerstörung der Zellen im Reaktor führen kann. Es ist somit die Bereitstellung einer physiologischen Gewebecompliance (Dehnbarkeit des Gewebes) im dreidimensionalen Environment erforderlich, was mit bisherigen Systemen nicht möglich ist.

Zum Weiteren muss ein solcher Bioreaktor die Möglichkeit bieten, die Entwicklung eines solchen Blutgefäßnetzes oder die Entwicklung des Gewebes/Gewebeäquivalents funktionell und morphologisch beurteilen zu können, wenn möglich kontinuierlich mit noninvasiven Methoden. Bei vielen bisherigen Bioreaktoren ist es nicht möglich, ein umfassendes optisches oder funktionelles Monitoring mit mehreren Modalitäten durchzuführen, und gleichzeitig noch den Druck und den Fluß der Perfusion zu messen. Somit können Vorgänge wie die Vaskularisierung eines Gewebes ausgehend von einer Blutgefäßwand oder eines mittels Engineering hergestellten Blutgefäßwandäquivalents bislang nicht oder nur schwer mittels optischer Geräte, z.B. Laser Scanning Mikroskopie (Konfokalmikroskop) oder funktionell mittels ESR (Elektronspinresonanz)-Spektroskopie, benchtop MRI (Kerspintomografie) Geräten visualisiert bzw. überwacht werden, weil sie aufgrund ihrer Größe nicht in den Schacht dieser Geräte eingerührt werden können. Dieses ist aber notwendig, um physikalische, hydrodynamische, Kultivierungsparameter und Wachstumsfaktorwirkmigen auf die Ausbildung eines funktionellen Gefäßnetzwerks in vitro beurteilen zu können.

Ein spezifisches Problem bei der Einbindung eines Gefäßes, eines Gefäßkonstrukts oder einer Gefäßwand, eines Gefäßkonstrukt in einen Bioreaktors mit kleinen Dimensionen, der sich für die o. g. Untersuchungen eignet, ist die kontrollierte Bereitstellung eines relativen Überdruck in dem Gefäßkompartiment - zumindest während der Pulsation (Systole) - gegenüber den "Interstitium" wie bei einem natürlichen Gefäß und Gewebe. Besonders schwierig ist dies, wenn ein Gefäßwandäquivalent eingespannt werden soll, da seine Oberfläche gleichzeitig beobachtbar bleiben soll. Aufgrund der Fragilität derartiger Konstrukte besteht das Problem darin, eine Abdichtung zu gewährleisten, die eine zumindest kurzzeitige Druckerhöhung in dem Gefäßkompartiment zulässt (durch den Puls bei einer pulsatorischen Perfusion). Ein Modell, das dieses auf engem Raum erlaubt, kann für viele unterschiedliche Fragestellungen aus den Bereichen Regeneration und Tissue Engineering, Angiogenese, Kreislauf- und Stoffwechselforschung, Onkologie (Metastasierung) u.v.a.m. genutzt werden.

Aufgabe der Erfindung ist es, einen Bioreaktor bereit zu stellen, welcher die Nachteile des Standes der Technik überwindet.

Die Aufgabe der Erfindung wird durch die Merkmale des Anspruches 1 gelöst.

Erfindungswesentlich ist insbesondere, dass der Bioreaktor zu großen Anteilen elastische Wandungen hat, röhrenförmig aufgebaut ist und im Innenraum ein Blutgefäß, eine Blutgefäßwand oder ein entsprechendes Konstrukt ("Blutgefäßäquivalent") so eingebracht werden kann, dass es in der Längsachse des Bioreaktors angeordnet mit einem relativen Überdruck gegenüber dem Umgebungsdruck (im Bioreaktor) perfundiert werden kann und zudem über durchsichtige Sichtfenster die Möglichkeit besteht, es mit optischen Vergrößenmgsgeräten, z.B. Laser Scanning Mikroskopen zu beobachten.
Alle Anschlüsse und Öffnungen für das Einbringen und Manipulieren von Geweben, Gerüsten, oder Gerüst-Zellkombinationen werden von den Kopfseiten aus eingeführt. Der Gesamtdurchmesser überschreitet bevorzugt 17 mm, besonders bevorzugt 13 mm nicht, so dass der Bioreaktor während des laufenden Betriebs im Schacht von benchtop MRI- und ESR-Geräten untersucht werden kann. Die Perfusion erfolgt über ein selbstregulierendes Perfusionssystem, welches das Perfusionsmedium transportiert und die Einstellung unterschiedlicher Perfusionsmodi (z.B. Pulsation, Frequenz etc.) erlaubt.

Es gibt zwei Varianten: in Variante 1 ist die Blutgefäßwand bzw. das Blutgefäßäquivalent mit seiner (endothelialen) Innenseite zur Sichtscheibe hin offen angeordnet. Die perfundierbare Kammer wird somit durch die Blutgefäßwand/Blutgefäßwandäquivalent, durch die Sichtscheibe (die ebenfalls aus elastischem Material gefertigt sein kann) und durch eine umlaufende starre Andruckfläche gebildet. Die Abdichtung der Kammer gegenüber dem übrigen Kammervolumen wird dadurch erzielt, dass das Blutgefäßwand/Blutgefäßwandäquivalent mit einem elastischen Formkörper (z.B. elastischer Schaumstoff) gegen die umlaufende Andrückfläche gepresst wird. Der Andruck im Bereich der umlaufenden Andruckfläche kann dadurch unterstützt werden, dass ein zweiter, zur Andruckfläche kongruenter Rahmen auf der Unterfläche des Blutgefäßwand/Blutgefäßwandäquivalents platziert wird und zum Weiteren, dass der elastische Formkörper biphasisch konstruiert wird, d.h. mit einem geringer elastischen umlaufenden Rand, der das Blutgefäßwand/Blutgefäßwandäquivalent im Bereich der umlaufenden Andruckfläche unterstützt und einem höher elastischen Kern, der die druckbedingten Auslenkungen des mittigen Bereichs des Blutgefäßwand/Blutgefäßwandäquivalents begünstigt. Durch diese Maßnahmen wird umlaufend eine Abdichtung geschaffen, die zwar nicht vollständig flüssigkeitsdicht ist, aber unter Schonung des Blutgefäßwand/Blutgefäßwandäquivalent eine Abdichtung soweit bereitstellt, dass während der Pulsphase kurzzeitig eine messbare Druckerhöhung in der perfundierbaren Kammer auftritt (bis in die Höhe physiologischer und pathologischer Systolenwerte).
Dieser grundsätzliche Aufbau bietet die Möglichkeit, zwischen BlutgefÄßwand/Blutgefäßwandäquivalent und elastischem Formkörper ein beliebiges Gewebe oder Gewebeäquivalent zu platzieren, in das kleine Blutgefäße von der Blutgefäßwand/Blutgefäßwandäquivalent einsprossen können, was mit den o. g. Bildgebungs- und Messmethoden beobachtet werden soll. Es kann auch der gesamte restliche Bioreaktorhohlraum statt des elastischen Formkörpers mit einem solchen Gewebe oder Gewebeäquivalent ausgefüllt werden, so dass allein die elastische Bioreaktorwand durch ihren Druck die Blutgefäßwand/Blutgefäßwandäquivalent an die umlaufende Andrückfläche presst und so die Abdichtung erzielt wird.
Das Perfusionsmedium kann die perfundierbare Kammer über den vorbezeichneten Abfluss verlassen und zusätzlich durch das Gewebe (Perfusion i.e.S., z.B. durch gewachsene Kapillaren oder künstlich angelegte Kanäle im Gewebsstück) hindurch in das von Schaumstoff / elastisches Formmaterial ausgefüllte Restvolumen des Bioreaktors, von wo es über einen zusätzlichen Abfluss aus dem Bioreaktor austritt. Damit kann eine Gewebeeinheit funktionell und anatomisch simuliert werden, die aus einem versorgenden Blutgefäß und einem beliebigen, anhängenden, versorgten Gewebe besteht.
So wird beispielsweise eine pulsatile Perfusion über das Gewebe gegen den Schaumstoff) bzw. die elastische Wandung fortgeleitet und es können mechanische bzw. hydrodynamische Belastungen auf das Gewebe einwirken. Der wesentliche Unterschied zu bisherigen Lösungen liegt darin, dass diese Perfusiondynamik in einem elastischen Umfeld erfolgt und durch Einstellung der Elastizität der Kammerwand die Compliance natürlicher Blutgefäße und Gewebe in physiologischer und pathologischer Situation modelliert werden können.
In die Wand und/oder die umlaufende Andrückfläche können die Anschlüsse für Monitoringsysteme integriert werden. Das Monitoring (Überwachung und Kontrolle) erfolgt über ein Sondensystem, welches Stoffkonzentrationen und physikalische bzw. chemische Kenngrößen wie z.B. O₂- und CO₂-Konzentration, Druck in der Kammer und im restlichen Bioreaktor, Sauerstoffpartialdruck, pH-Wert, Fließgeschwindigkeit und Temperatur überwacht. Die Dehnung der elastischen Wände kann mit Dehnungsmessstreifen überwacht werden. Das Monitoring trägt außerdem aktiv zur Regulierung der Wachstumsbedingungen im Bioreaktorsystem bei, da es als Sensorik in einen Regelkreis eingebunden ist.

In der zweiten Variante des erfindungsgemäßen Bioreaktors besteht die perfundierbare Kammer aus einem Blutgefäß oder Blutgefäßäquivalent, das an der Sichtscheibe entlang geführt wird und durch zwei Anschlüsse Zu- und Abfluss erhält, Dadurch kann es ebenfalls mit CLSM, MRI (Kernspintomografen) oder ESR (Elektronspinresonanz-Geräten) beobachtet werden, allerdings nicht durch direkte Aufsicht auf die endotheliale Oberfläche. Statt einer separaten Sichtscheibe kann auch der gesamte elastische Anteil der Bioreaktorwand aus einem klar durchsichtigen elastischen Material gefertigt sein.

Die Form und Anordnung aller Bauelemente erlaubt folglich die Herstellung von besonders kleinen bzw. dünnen Bioreaktoren, welche in speziellen Anwendungsfällen an räumlich limitierende Vorgaben gebunden sind, z.B. unter einem Laser Scanning Mikroskop oder im Schacht von ESR- Geräten und benchtop(Tischgerät)-MRIs. Alle eben aufgeführten Vorteile der Erfindung tragen somit deutlich zur Verbesserung bisheriger Bioreaktorsysteme, der Wachstumsverhältnisse in Bioreaktoren und der Qualität gezüchteter Gewebekonstrukte bei und haben positive Auswirkungen auf das Tissue Engineering im Allgemeinen und Speziellen.
Der Bioreaktor kann für den einmaligen Gebrauch konzipiert sein.

Die Unteramsprüche geben vorteilhafte Ausgestaltungen der Erfindung an, ohne diese damit zu beschränken.

Bevorzugt ist weiterhin, dass das elastische Teilsegment mehr als 50% der inneren Oberfläche der Reaktorwandung 6 beträgt, besonders bevorzugt mehr als 75%.
Bevorzugt ist weiterhin, dass die Elastizität dieses Materials des Teilsegmentes so eingestellt werden kann, dass die Dehnung von Gewebe bzw. Gewebeäquivalent in der Hülle durch den im Inneren erzeugten Perfusionsdruck den physiologischen oder pathologischen Werten der Gewebecompliance des herzustellenden Gewebes entspricht. Bevorzugt ist, dass sein Gesamtdurchmesser 17 mm, besonders bevorzugt 13 mm nicht überschreitet und im Schacht von ESR(Elektronspinresonanz)-Geräten und MRI(Kernspintomografen)-Geräten untersucht werden kann.
Bevorzugt ist, dass er mindestens auf einer Kopfseite über eine verschließbare, besonders bevorzugt mit Schraubverschluß 12 verschließbare Öffnung 4 verfügt, über die die Einführung von Geweben, Blutgefäßen, Äquivalenten und Formkörpern erfolgt. Bevorzugt ist, dass die perfundierbare Druck-Kammer 9 und Sichtscheibe 11 so dimensioniert sind, dass das Gefäß-Gewebsstück 18 mit optischen Vergrößerungsapparaten, speziell Fluoreszenzmikroskopen und konfokalen (Laser Scanning) Mikroskopen, betrachtbar sind.

Bevorzugt ist weiterhin, dass als elastischer Formkörper ein biphasisches elastisches Material 20 verwendet wird, das in seinen zentralen Anteilen 20.2 eine höhere Elastizität aufweist als im Randbereich 20.1, so dass die Auslenkung des Gefäß-Gewebsstücks durch den Perfusionsdruck in seinem zentralen Anteil gegenüber dem Randbereich verstärkt ist und eine Unterstützung des Anpressdrucks des seitlichen Randbereichs des Konstrukts erreicht wird.
Bevorzugt ist weiterhin, dass der Bioreaktor im Inneren unmittelbar neben und entlang des Sichtfensterbereichs eine Einspannvorrichtung enthält, in die zwischen zwei Schlauchanschlüssen 16.2 und 16.1 ein Blutgefäß, ein Blutgefaßäquivalent oder ein Gerüst für ein mittels Tissue engineering herzustellendes Blutgefäß 21 eingespannt werden kann, so dass das Blutgefäß, das Blutgefäßäquivalent oder das Gerüst für ein mittels Tissue engineering herzustellendes Blutgefäß 21 durch das Sichtfenster von außen und mittels optischer Geräte, vorzugsweise konfokale Laser Scanning Mikroskopie, beobachbar ist.

Bevorzugt ist weiterhin, dass die perfundierbare Druck-Kammer oder das Blutgefäß, Blutgefaßäquivalent oder das Gerüst für ein mittels Tissue engineering herzustellendes Blutgefäße 21. mit Kulturmedium oder Blut oder einer Mischung von beidem perfundierbar ist.
Bevorzugt ist weiterhin, dass dieser als Einheit in Verbindung mit einem selbstregulierenden pulsatorischen Perfusionssystem betreibbar ist.

Bevorzugt ist weiterhin, dass über die Perfusionsdynamik, insbesondere mittels pulsatorischer Perfusion mit einem selbststeuernden Perfusionssystem, mechanische Belastungen in Größenordnungen physiologischer Kräfte, insbesondere Blutdruckwerte des gesamten menschlichen Gefäßsystems, auf das Gewebe ausübbar sind.
Bevorzugt ist weiterhin, dass er im Innenraum resorbierbare oder nicht resorbierbare Hohlfasersysteme, Leitungssysteme oder Gerüststrukturen enthält, über die das Kulturmedium verteilt wird, welche wiederum das herzustellende Gewebe oder Gewebeäquivalent ernähren.

### Erläuterung der Erfindung an Ausführungsbeispielen

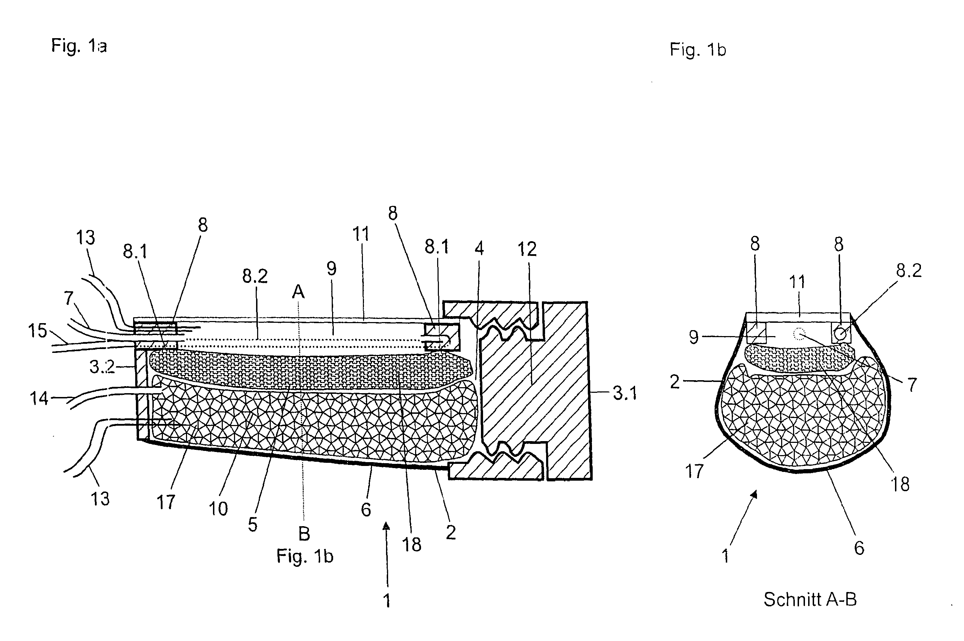

### Bespiel 1 - Bioreaktor nach Figur 1 a und 1b

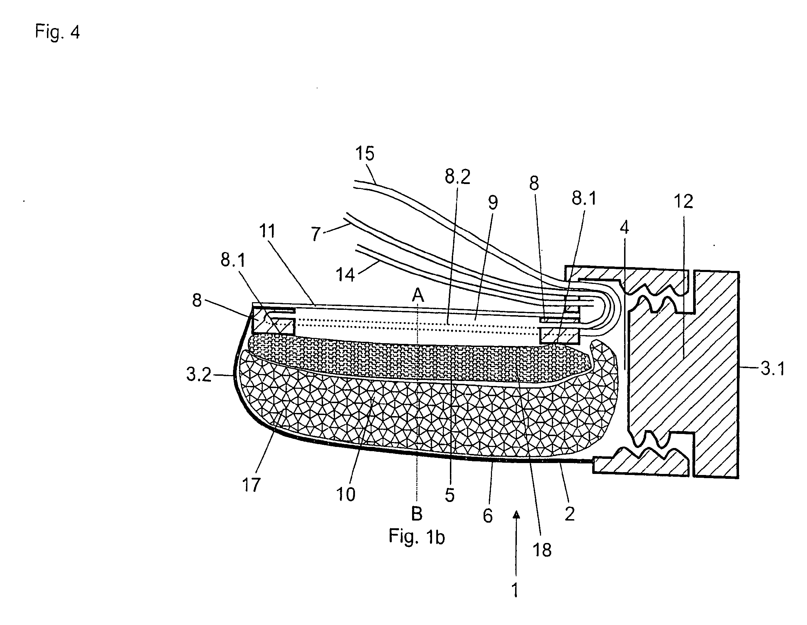

Der Bioreaktor 1 dient zur Herstellung und/oder Kultivierung eines menschlichen oder tierischen Blutgefäßes und/oder eines menschlichen oder tierischen Gewebe (Gefäß-Gewebsstück). Der Bioreaktor 1 besitzt einen rohrförmigen, hohlen Grundkörper 2, mit zwei Stirnseiten 3.1 und 3.2.
An der Stirnseite 3.1 ist eine wieder verschließbare, flüssigkeitsdichte Öffnung für Beladung und Zusammenbau 4 angeordnet, welche insbesondere zum Eintrag des Konstrukts 18 in den Innenraum 5 des Reaktors dient. Die Öffnung 4 ist als üblicher, flüssigkeitsdichter Schraubverschluss 12 ausgebildet.
Im Innenraum 5 ist eine perfundierbare Druck-Kammer (perfundierbare Kammer) 9 angeordnet, die gleichzeitig ein Teil des Innenraums 5 ist, die etwa parallel zur Reaktorlängsacbse des Reaktors 1 angeordnet ist und die hin zur Reaktorachse offen ist. In den Teil der perfundierbaren Druck-Kammer (perfundierbare Kammer) 9, der im Bereich der Stirnseite 3.2 angeordnet ist, mündet der Zufluss 7 in die Druck-Kammer (perfundierbare Kammer) 9. In dem Bereich sind außerdem Sonden (z.B. Druck, Fluß, pH) 13 des Monitoringsystems angeordnet, die in die Druck-Kammer (perfundierbare Kammer) 9 ragen.
Die Druck-Kammer (perfundierbare Kammer) 9 besitzt eine umlaufende Andrückfläche 8.1 der Halterung 8, an welche flüssigkeitsdichtend das Konstrukt (Gewebestück) 18, hier ein Gefäßwandäquivalent gedrückt ist. Das Konstrukt 18 bildet somit ein perfundierbare Trennwand zwischen der Druck-Kammer (perfundierbare Kammer) 9 und der Konstrukt-Kammer 10, wobei das Konstrukt dabei an die Andrückfläche 8.1 angedrückt ist.
Einen Teil der Druck-Kammer (perfundierbare Kammer) 9 bildet das Monitoringfenster 11, welches als transparente Sichtscheibe ausgebildet ist.
Im Innenraum 5 befindet sich außerdem eine Konstrukt-Kammer 10, die etwa parallel zur Reaktorlängsachse angeordnet ist und hin zur Reaktorachse offen ist bzw. im befüllten Zustand, wie in Figur 1 dargestellt, durch das Konstrukt verschlossen ist.
In den Teil der Konstrukt-Kammer 10, der im Bereich der Stirnseite 3.2 angeordnet ist, mündet der Abfluss(Abfluss aus Reaktorraum) 14 in die Konstrukt-Kammer 10.. Der verbliebene Hohlraum der Konstrukt-Kammer 10, d. h. der Raum, welcher nicht durch das Konstrukt ausgefüllt ist, ist, wie in Figur 1 dargestellt, durch ein elastisches Formteil (elastischer Formkörper) 17 ausgefüllt.
Ein Teil der Reaktorwandung 6, welcher ein Teil der Konstrukt-Kammer 10 ist, besteht aus elastischem Material, beispielsweise elastische Silikonfolie.
Der rohrförmige Grundkörper 2 ist der Art dimensioniert, so dass er in die Öffnung eines ESR- oder benchtop MRI- Gerätes einschiebbar ist. Die Stirnseiten 3.1. und 3.2 sind somit im Durchmesser jeweils nicht größer als 13 mm (oder 17mm für ESR).
Im Figur 1 dargestellten Zustand wird in den Bioreaktor 1 über den Zufluss 7 ein Kulturmedium, Blut, oder dgl. mit Überdruck in die Druck-Kammer 9 eingebracht. Ein Teil dieses flüssigen Mediums durchströmt das perfundierbare Konstrukt und gelangt somit in den Bereich der Konstrukt-Kammer 10. Über den Abfluss 14 und 15 wird flüssige Medium aus dem Bioreaktor geführt. Das flüssige Medium wird Damit einer Einheit zur Druckerzeugung, beispielsweise einer peristaltischen Pumpe, zugeführt. Durch die Einheit zur Druckerzeugung sind auch pulsierende Zuflüsse generierbar, die dann pulsierend in der Druck-Kammer (perfundierbare Kammer) 9 anliegen. Dies bewirkt ein zumindest partielles Auslenken des Konstrukts hin zur Konstrukt-Kammer 10 und damit durch Verdrängen des Inhaltes der Konstrukt-Kammer 10 auch ein Ausdehnen der elastischen Reaktorwand 6 außen.
Auch andere Anordnungen sind bei demselben Grundprinzip möglich (Fig. 4).

### Beispiel 2 - Betrieb des teilelastischen Bioreaktors

Es kann nun ein vorbesiedeltes flaches Gefäßwandäquivalent 18, z.B. bestehend aus einem Gedüst mit mesenchymalen Zellen und Endothelzellen zur Simulation einer Gefäßwand auf die Größe der umlaufenden Andrückfläche 8.1 zugeschnitten und flach auf selbiger platziert werden (endotheliale Seite zur Sichtplatte 11 hin). Dadurch entsteht zwischen Glasplatte 11 und Gewebeäquivalent ein spaltförmiger Hohlraum 9 der dem perfundierbaren Kammervolumen entspricht und durch die Glasplatte 11 direkt von oben betrachtet werden kann. Die der Sichtplatte 11 zugewandte Seite des Gewebes entspricht in diesem Modell der Innenseite einer Blutgefäßwand. Die Sichtplatte 11 ermöglicht das direkte optische (z.B. durch Mikroskopie, Fluoreszenzmikroskopie, Laserscanning-Mikroskopie etc.) Monitoring der sich entwickelnden Oberfläche der Gefäßwand an der Grenzfläche zur Perfusionskammer 9 und auch ein non-invasives funktionelles Monitoring (z.B. durch ESR-Spektroskopie, MRI) des Gefäßwandäquivalents während des Reaktorbetriebs.
Auf die Unterfläche des Gefäßwandäquivalents 18 wird ein Gewebeäquivalent 19, z.B. ein Scaffold mit mesenchymalen Zellen eines Zielgewebes, z.B. Muskel, Fettgewebe oder Knochen platziert. Der restliche Hohlraum des Bioreaktors 1 wird mit einem elastischen Schaumstoff gefüllt. Bei einer pulsatorischen oder anderen Druckperfusion wird das Gewebeäquivalent /Gefäßwandäquivalent nach Bedarf physiologischen oder pathologischen Drücken und Auslenkungen unterworfen, die durch Druckmesssysteme im Inneren der Kammer messbar sind.

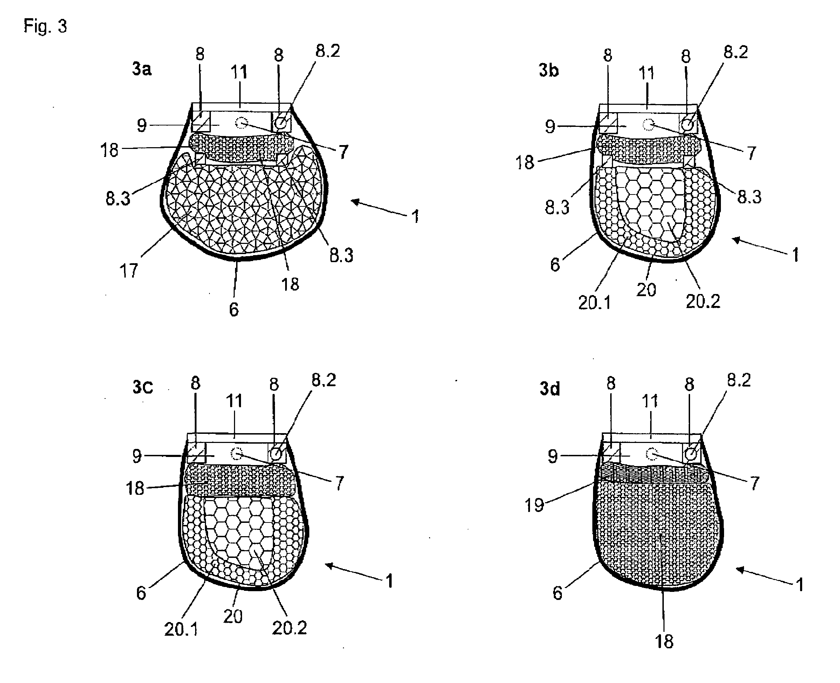

### Beispiel 3 - Maßnahmen zur Abdichtung an der umlaufenden Andrückfläche (Fig. 3)

Ein entsprechend dem Bioreaktorinnenraum geformte elastische Fixationsvorrichtung (z.B. ein geformtes Stück Schaumstoff) 17 wird in den Bioreaktorinnenraum eingeführt und drückt das Gewebsstück) 18 gegen den Rahmen 8, wodurch eine Abdichtung des perfundierbaren Kammervolumens 9 erreicht wird. Der Schaumstoff füllt somit den Hohlraum zwischen dem Gewebsstück 18 und der flexiblen Wandung 6 aus und fixiert das Gewebsstück 18 gleichzeitig auf dem Rahmen 8.
Zusätzlich kann hier noch ein fester oder elastischer Kunststoffrahmen (zusätzlich zwischengelegter Rahmen) 8.3 in der Ausdehnung des Rahmens an der Sichtplatte 11 zwischen Gewebsstück 18 und Schaumstoff 17 gelegt werden, so dass der Rand des Gewebsstück 18 zwischen zwei den Rahmen fixiert wird (Fig. 3a).

Als Alternative zu dem Schaumstoffeinsatz kann der Durchmesser des Bioreaktorinnenraums verringert werden, um das Gewebestück direkt zwischen Rahmen und elastischer Wandung zu fixieren, oder das Gewebestück oder Gewebeäquivalent 18 mit einer Gefäßwand oder einem Gefäßwandäquivalent 19 so groß zu gestalten, dass es den Hohlraum vollständig, also ohne Schaumstoff ausfüllt (Fig. 3d: Querschnitt durch Bioreaktor 1 ohne Formkörper, mit Gefäß oder Gefäßäquivalent und anhängendem Gewebe oder Gewebeäquivalent, den Bioreaktorraum vollständig ausfüllend).

Eine weitere Ergänzung und Einstellung der Compliance kann durch Varianten des elastischen Formkörpers (Schaumstoff) erreicht werden. So kann beispielsweise ein biphasischer elastischer Formkörper/Schaumstoff 20 eingesetzt werden, bei dem der Rand (feste Zone) 20.1 weniger elastisch ist als das Zentrum (Zone mit hoher Elastizität) 20.2. Damit wird erreicht, dass durch die pulsatorische Perfusion das Gewebsstück im Zentrum leichter ausgelenkt wird und am Rand besser fixiert und abgedichtet bleibt (Fig. 3b mit biphasischem Formkörper und zwischengelegtem Rahmen und Fig. 3c mit biphasischem Formkörper ohne zwisehengelegten Rahmen).

### Beispiel 4 - Zusätzliche Integration einer Vorrichtung zur Regulierung der Dehnbarkeit der perfundierbaren Kammer

Um die Dehnbarkeit ("Compliance") der perfundierbaren Kammer weiter anzupassen, kann die Sichtplatte wie folgt modifiziert werden: Hierzu wird sie mit einer mittigen, an die Größe des Rahmens angepassten Aussparung versehen und eine zweite Trägerplatte mit derselben Aussparung angefertigt. Nun kann eine hochelastische Membran zwischen beiden Trägerplatten eingeklebt werden und fungiert fortan als neues Teilstück dcr Wandung sowie als Regulator der Compliance, indem sie sich bei jedem Puls nach außen ausdehnt und anschließend wieder strafft. Diese Membran ist vorzugsweise durchsichtig, damit durch sie hindurch die perfundierte Gewebefläche beobachtet und mikroskopiert werden kann. Somit ist außer dem festen Rahmen die gesamte Kammerwandung aus elastischem Material aufgebaut.

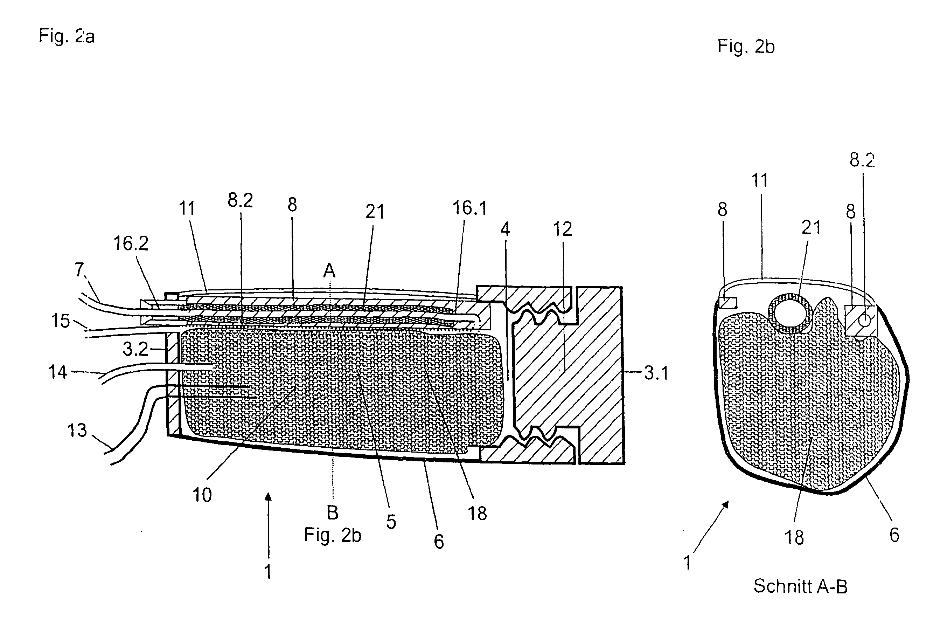

### Beispiel 5 - Variante mit röhrenförmigem Gefäß (Fig. 2a und b)

Eine Ausführungsvariante besteht darin, dass keine perfundierbare Kammer i.e.S. besteht, sondern eine schlanke Halterung 8 in die Kammerwandung integriert ist und die beiden Stirnseiten verbindet, an deren Enden jeweils Anschlüsse befestigt sind, die zum Anschluss eines Blutgefäßes oder Blutgefäßäquivalents 21 dienen. Der Anschluss am Zufluss 7 muss so gestaltet werden, dass das Gefaß/Gefaßäquivalent 21 durch die große Öffnung 4 steril eingeführt und an der Stirnseite 3.2 angekuppelt werden kann. Dazu ist die Stirnseite 3.2 mit einer kleineren Öffnung mit einen Flansch versehen, durch den von außen eine Kupplung 16.2 mit einer Schlaucholive eingeführt werden kann, auf der das Gefäß/Gefäßäquivalent 21 fixiert wird. Diese Kupplung 16.2 wird z.B. mit Luer-Lock Prinzip flüssigkeitsdicht an dem Flansch befestigt und fixiert das Gefäß/Gefäßäquivalent 21. Danach wird das Gefäß/Gefäßäquivalent 21 an dem Anschluss 16.1 (z.B. Schlaucholive) befestigt. Die Leitung 8.3 für den Abfluss 15 wird wie bei der erstbeschriebenen Variante im Rahmen 8 oder an ihm entlang geführt. Somit wird ein röhrenförmiges Konstrukt perfundiert, alle anderen Merkmale der Kammer bleiben erhalten. Da nun die Perfusionsfläche nicht mehr eingesehen werden kann, sind optische Methoden für das Monitoring gegenüber der Erstvariante erschwert. Es kann auf diese Art und Weise jedoch ein Blutgefäß mittels Tissue Engineering hergestellt werden oder simuliert werden, dass in direktem Kontakt zu einem versorgten Gewebeabschnitt (Konstrukt, Gewebsstück) 18 steht. Dadurch können zum Beispiel die Bedingungen untersucht werden, unter denen es zum Einwachsen von Aussprossungen (kleinen Blutgefäßen) von dem zentralen Gefäß in das anhängende Gewebe (Konstrukt, Gewebsstück) 18 kommt. Wenn man statt mittels Tissue engineering hergestellter Konstrukte menschliche oder tierische Blutgefäße oder Gewebe nimmt, kann man auch physiologische oder pathologische Prozesse in vitro untersuchen, die bislang nur Tierversuchen vorbehalten waren. Dies gilt vorzugsweise für pathologische und physiologische Prozesse an Gefäßen oder am Kreislaufsystem, für die Adipositasforschung und für die Testung pharmakologischer Substanzen, bei denen die Interaktionen zwischen Blutgefäßen und Gewebe eine Rolle spielen.
Im Ausführungsbeispiel gemäß den Figuren 2a und 2b ist das Monitoringfenster 11 als durchsichtige Folie ausgeführt.

### Beispiel 6 - Anschluss und Betrieb des selbstregulierenden pulsatorischen Perfusionssystems

Ein selbstregulierendes pulsatorisch arbeitendes Perfusionssystem wird mit dem Bioreaktor verbunden und dient zur Simulation physiologischer oder experimenteller Druckverhältnisse.

### Beispiel 7 - Monitoring

Ein umfassendes Monitoringsystem wird mit den Anschlüssen des Rahmens auf der Glasscheibe des Bioreaktors verbunden und erlaubt die Überwachung wichtiger Parameter (O₂, SpO₂, CO₂, pH, Druck, Temperatur, Viskosität, Fließgeschwindigkeiten etc.) in Echtzeit.

### Beispiel 8 - Anwendungen

Anwendungsmöglichkeiten für den erfindungsgemäßen Bioreaktor ergeben sich überall dort, wo die Interaktionen zwischen Gesäß und Stroma bzw. mesenchymalen oder anderen Gewebe eine Rolle spielen. Dies sind viele Bereiche neben den bereits skizzierten Anwendungen in der regenerativen Medizin und beim Tissue engineering. Das System kann wie in den Vorbeispielen skizziert, analog zu den Gewebeäquivalenten bzw. artifiziellen Geweben genauso mit natürlichen, explantierten Geweben und Gefäßen betrieben werden. Damit ergibt sich ein breiter Anwendungsbereich. Dies können beispielsweise grundlagenorientierte Untersuchungen insbesondere in der Erforschung von Kreislauferkrankungen sein, aber auch vieler Stoffwechselstörungen, wie z.B. Adipositas, bei der das Wechselspiel von Gefäßen und Fettzellen eine wesentliche Rolle spielt. Weiterhin kann es als Metastasenmodell in der onkologischen Forschung nützlich sein. Fragen zur Wundheilung können damit beantwortet werden, und es kann auch als Angiogenesentodell in der Grundlagenforschung verwendet werden. Ein wesentlicher Zweig ist auch die Anwendung in der Testung von Pharmaka, z.B. die Testung des Übertritts von Pharmaka in das Interstitium oder andere Fragestellungen.
Hier und bei anderen Anwendungen kann es auch als Ersatz für Tierversuche angewendet werden.

## Patentansprüche

1. Perfundierbarer Bioreaktor zur Herstellung und/oder Kultivierung eines menschlichen oder tierischen Blutgefäßes und/oder eines menschlichen oder tierischen Gewebes, wobei der Bioreaktor (1) zumindest besitzt:
- einen rohrförmigen Grundkörper (2), mit zwei Stirnseiten (3.1) und (3.2),
- eine wieder verschließbare, flüssigkeitsdiclate Öffnung (4), die an einer Stirnseite angeordnet ist,
- einen Innenraum (5),
- eine Reaktorwandung (6),
- zumindest einen Zufluss (7) und zumindest einen Abfluss (14) für ein flüssiges Medium,
- eine Halterung (8) für das einzubringende Konstrukt,
**dadurch gekennzeichnet, dass**
- im Innenraum eine perfundierbare Druck-Kammer (9) angeordnet ist, die parallel zur Reaktorlängsachse angeordnet ist,
- zumindest ein Zufluss (7) und ein Abfluss (15) in die Druck-Kammer (9) münden und die Druck-Kammer (9) hin zur Reaktorachse offen ist,
- im Innenraum (5) eine Konstrukt-Kammer (10) angeordnet ist, die parallel zur Reaktorlängsachse angeordnet ist und hin zur Reaktorachse offen ist,
- zumindest ein Abfluss (14) in die Konstrukt-Kammer (10) mündet,
- das in den Reaktor (1) eingebrachte Konstrukt als Trennwand zwischen der perfundierbaren Druck-Kammer (9) und der Konstrukt-Kammer (10) anordnenbar ist und
- zumindest ein Teilsegment der Reaktorwandung (6), welches zumindest ein Teil der Konstrukt-Kammer (10) ist, aus elastischem Material besteht.

2. Bioreaktor nach Anspruch 1, **dadurch gekennzeichnet, dass** in der Reaktorwandung (6), bevorzugt im Bereich der Druck-Kammer (9), ein transparentes, starres oder elastisches Monitoringfenster (11) angeordnet ist oder die Reaktorhülle im Bereich der Druck-Kammer (9) oder ggf. auch darüber hinaus aus transparentem Material gefertigt ist.

3. Bioreaktor nach Anspruch 1, **dadurch gekennzeichnet, dass** der Bioreaktor mit relativen Überdruck betreibbar ist, wobei der anliegende Druck in der Druck-Kammer (9) größer ist und als der in der Konstrukt-Kammer (10).

4. Bioreaktor nach Anspruch 1, **dadurch gekennzeichnet, dass** die Halterung (8) zumindest eine Andrückfläche (8.1) besitzt, wobei das Konstrukt an die Andrückfläche (8.1) andrückbar ist.

5. Bioreaktor nach Anspruch 1, **dadurch gekennzeichnet, dass** die Halterung (8) zumindest zwei Anschlüsse (16.1) und (16.2) besitzt, zwischen denen ein röhrenförmiges Blutgefäß, Blutgefäßäquivalent oder Gerüst für ein mittels Tissue engineering herzustellendes Blutgefäß (21) einspannbar ist.

6. Bioreaktor nach Anspruch 1, **dadurch gekennzeichnet, dass** das physikalische Druck-Belastungsregime, welches im Innenraum erzeugbar ist, dem physikalischen Druck-Belastungsregime entspricht, welches unter normalen physiologischen oder pathologischen Bedingungen im lebenden menschlichen oder tierischen Organismus auf die hergestellten Gewebe, Gewebeäquivalente, Blutgefäße oder Blutgefäßnetze einwirkt.

7. Bioreaktor nach Anspruch 1 **dadurch gekennzeichnet, dass** alle Zu-und Abflüsse und Anschlüsse für Sonden an den Stirnseiten in den Reaktor hineingeführt werden.

8. Bioreaktor nach Anspruch 1 **dadurch gekennzeichnet, dass** er mindestens auf einer Kopfseite über eine verschließbare, besonders bevorzugt mit Schraubverschluß (12) verschließbare Öffnung (4) verfügt, über die die Einführung von Geweben, Blutgefäßen, Äquivalenten und Formkörpern erfolgt.

9. Bioreaktor nach Anspruch 1, **gekennzeichnet dadurch, dass** zwischen dem elastischen Formkörper und dem Konstrukt ein zweiter zum Rahmen (8) kongruenter Rahmen (8.3) eingelegt ist, der den Anpressdruck des Konstrukts im Randbereich unterstützt.

10. Bioreaktor nach Anspruch 1, **gekennzeichnet dadurch, dass** auf der Stirnseite (3.2) eine zweite kleine, flüssigkeitsdichte Öffnung vorliegt, über die ein Blutgefäße, ein Blutgefaßäquivalent oder das Gerüst für ein mittels Tissue engineering herzustellendes Blutgefäß unter sterilen Bedingungen an den Zufluss (7) gekuppelt werden kann, besonders bevorzugt mit einem modifizierten Luer-Lock-Anschluss.

11. Bioreaktor nach Anspruch 1, **dadurch gekennzeichnet, dass** er ohne jede metallische Bauteile auskommt.

12. Verfahren zur Herstellung von menschlichen oder tierischen Geweben, Gewebeäquivalenten, Blutgefäßen oder Blutgefäßnetzen unter Verwendung eines Bioreaktors gemäß zumindest eines der Ansprüche 1 bis 11.

13. Verfahren zur Herstellung eines artifiziellen, versorgenden, mittels Tissue Engineering hergestellten Blutgefäßsystems unter Verwendung eines Bioreaktors gemäß zumindest eines der Ansprüche 1 bis 11, wobei das Verfahren zumindest folgende Schritte mit umfasst: das Konstrukt wird in den Innenraum des Bioreaktors eingebracht und der verbliebene Hohlraum des Innenraumes wird mit flüssigem Nähr-Medium gefüllt und über Zufluss und Abfluss mit einem Näher-Medium perfundiert.

14. Verwendung von einem Bioreaktor gemäß zumindest einem der Ansprüche 1 bis 11 für die Herstellung von menschlichen und tierischen Geweben für die klinische und therapeutische Nutzung.

15. Verwendung eines Bioreaktors gemäß zumindest einem der Ansprüche 1 bis 11 für die Testung pharmakologischer Substanzen, insbesondere auf dem Gebiet der Kreislauf- und Adipositasforschung, Wundheilung und Onkologie.

## Claims

1. Perfusable bioreactor for the production and/or culturing of human or animal blood vessels and/or human or animal tissue, with the bioreactor (1) incorporating the following minimum components:
- a tubular base body (2) with two front faces (3.1) and (3.2);
- a resealable liquid-tight orifice (4) located on one of the front faces;
- an internal space (5);
- a reactor cladding (6);
- at least one inlet (7) and one outlet (14) for liquid media;
- a retainer (8) for the construct to be inserted;
**characterised by**
- the interior space holding a perfusable pressure chamber (9) that is aligned in parallel with the longitudinal axis of the reactor;
- at least one inlet (7) and one outlet (15) terminating inside the pressure chamber (9) and the pressure chamber (9) being open toward the reactor axis;
- the interior space (5) holding a construct chamber (10) that is aligned in parallel with the longitudinal axis of the reactor, and opens toward the reactor axis;
- at least one outlet (14) terminating into the construct chamber (10);
- the possibility to use the construct inserted into the reactor (1) as dividing wall between the perfusable pressure chamber (9) and the construct chamber (10);
- at least one partial segment of the reactor cladding (6), which also constitutes a part of the construct chamber (19), being composed of elastic material.

2. Bioreactor according to claim 1, **characterised by** the reactor cladding (6) being fitted with a transparent rigid or elastic monitoring window (11), preferably in the area of the pressure chamber (9), or by the reactor shell adjacent to the pressure chamber (9) and, where appropriate beyond this area, being made up of transparent material.

3. Bioreactor according to claim 1, **characterised by** the bioreactor allowing for operation at relative overpressure, with the actual pressure inside the pressure chamber (9) exceeding the pressure inside the construct chamber (10).

4. Bioreactor according to claim 1, **characterised by** the retainer (8) having at least one pressure surface (8.1), with the construct being pushable against the pressure surface (8.1).

5. Bioreactor according to claim 1, **characterised by** the retainer (8) having at least two connections (16.1) and (16.2), with the possibility to clamp a tubular blood vessel, blood vessel equivalent, or scaffold for a blood vessel to be tissue-engineered between connections.

6. Bioreactor according to claim 1, **characterised by** the maximum physical pressure-load regime in the internal space corresponding to the physical pressure-load regime that acts on the engineered tissues, tissue equivalents, blood vessels or blood vessel network under normal physiologic or pathologic conditions inside the living human or animal organism.

7. Bioreactor according to claim 1, **characterised by** any inlets, outlets, and probe connections on the front faces terminating inside the reactor.

8. Bioreactor according to claim 1, **characterised by** the reactor having a sealable orifice (4) (preferably with screw plug [12]) on at least one head end, which will be used to insert tissue, vessels, equivalents, and moulds.

9. Bioreactor according to claim 1, **characterised by** a second frame (8.3), which is congruent with the frame (8) and serves to support the surface pressure of the construct in the periphery, being inserted between the elastic mould and the construct and.

10. Bioreactor according to claim 1, **characterised by** the front face (3.2) having a second small, liquid-tight orifice allowing for a blood vessel, blood vessel equivalent, or scaffold for a blood vessel to be tissue-engineered, to be coupled to the inlet (7), in particular by way of a modified Luer taper.

11. Bioreactor according to claim 1, **characterised by** the absence of any metal components.

12. Method of producing human or animal tissues, tissue equivalents, blood vessels or blood vessel networks using a bioreactor according to at least one of claims 1 to 11.

13. Method of producing an artificial afferent tissue-engineered blood vessel system using a bioreactor according to at least one of claims 1 to 11, with the method comprising at least the following operations: The construct is brought into the internal space of the bioreactor, with the remaining blank volume of the internal space filled with liquid culture medium and perfused with culture medium via the inlet and outlet.

14. Operation of a bioreactor according to at least one of claims 1 to 11 for the production of human and animal tissues for clinical and therapeutic use.

15. Operation of a bioreactor according to at least one of claims 1 to 11 to test pharmacological compounds, in particular in the domains of circulation and obesity research, wound healing, and oncology.

## Revendications

1. Bioréacteur à perfusion pour la fabrication et/ou la culture d'un vaisseau sanguin humain ou animal et/ou d'un tissu humain ou animal, ce bioréacteur (1) comprenant au moins :
- un corps principal (2) tubulaire, comportant deux faces terminales (3.1) et (3.2),
- une ouverture (4) refermable étanche aux liquides, située sur une des faces terminales,
- un espace interne (5),
- une paroi de réacteur (6),
- au moins une entrée (7) et au moins une sortie (14) pour un milieu liquide,
- un dispositif de fixation (8) pour le tissu reconstitué à introduire dans le réacteur,
**caractérisé en ce que**
- dans l'espace interne se trouve une chambre de pression (9) à perfusion, qui est parallèle à l'axe longitudinal du réacteur,
- au moins une entrée (7) et une sortie (15) débouchent dans la chambre de pression (9) et la chambre de pression (9) est ouverte sur l'axe du réacteur,
- dans l'espace interne (5) se trouve une chambre de reconstitution tissulaire (10), qui est parallèle à l'axe longitudinal du réacteur et ouverte sur l'axe du réacteur,
- au moins une sortie (14) débouche dans la chambre de reconstitution tissulaire (10),
- le tissu reconstitué introduit dans le réacteur (1) peut être placé comme paroi de séparateur entre la chambre de pression (9) à perfusion et la chambre de reconstitution tissulaire (10) et
- au moins un segment partiel de la paroi du réacteur (6), qui est
au moins une partie de la chambre de reconstitution tissulaire (10), est composé d'une matière élastique.

2. Bioréacteur d'après l'exigence 1, **caractérisé en ce que** dans la paroi du réacteur (6), en priorité dans le secteur de la chambre de pression (9), est située une fenêtre de contrôle (11) transparente, fixe ou élastique, ou la gaine du réacteur dans le secteur de la chambre de pression (9) ou le cas échéant également au-delà est fabriquée dans une matière transparente.

3. Bioréacteur d'après l'exigence 1, **caractérisé en ce que** le bioréacteur peut être utilisé avec une surpression relative, la pression appliquée dans la chambre de pression (9) étant supérieure à celle dans la chambre de reconstitution tissulaire (10).

4. Bioréacteur d'après l'exigence 1, **caractérisé en ce que** le dispositif de fixation (8) a au moins une surface de pression (8.1), le tissu reconstitué pouvant être pressé contre la surface de pression (8.1).

5. Bioréacteur d'après l'exigence 1, **caractérisé en ce que** le dispositif de fixation (8) a au moins deux raccordements (16.1) et (16.2), entre lesquels un vaisseau sanguin tubulaire, l'équivalent de vaisseau sanguin ou une structure pour un vaisseau sanguin (21) à fabriquer au moyen de l'ingénierie tissulaire, peut être serré.

6. Bioréacteur d'après l'exigence 1, **caractérisé en ce que** le régime charge de pression physique, qui est généré dans l'espace interne, correspond au régime charge de pression physique, qui influence en conditions normales physiologiques ou pathologiques dans l'organisme vivant humain ou animal sur les tissus, les équivalents de tissu, les vaisseaux sanguins ou les réseaux de vaisseaux sanguins, qui sont fabriqués.

7. Bioréacteur d'après l'exigence 1, **caractérisé en ce que** toutes les entrées et les sorties ainsi que les raccordements pour les sondes aux faces terminales sont introduits dans le réacteur.

8. Bioréacteur d'après l'exigence 1, **caractérisé en ce qu'**il dispose au moins sur un côté en haut, d'une ouverture (4) verrouillable, surtout en priorité verrouillable avec une fermeture à vis (12), qui permet l'introduction de tissus,
de vaisseaux sanguins, d'équivalents et de corps moulés.

9. Bioréacteur d'après l'exigence 1, **caractérisé en ce que**, entre le corps moulé élastique et le tissu reconstitué un deuxième cadre (8.3) coïncident au cadre (8) est introduit, ce deuxième cadre soutenant la pression de serrage du tissu reconstitué dans la zone du bord.

10. Bioréacteur d'après l'exigence 1, **caractérisé en ce que** sur la face terminale (3.2) se trouve une deuxième petite ouverture, étanche aux liquides, par laquelle un vaisseau sanguin, un équivalent de vaisseau sanguin ou la structure pour un vaisseau sanguin à fabriquer au moyen de l'ingénierie tissulaire dans des conditions stériles peuvent être connectés à l'entrée (7), en particulier en priorité avec un raccord Luer-Lock modifié.

11. Bioréacteur d'après l'exigence 1, **caractérisé en ce qu'**il ne nécessite aucun composant métallique.

12. Procédé pour fabriquer des tissus, des équivalents de tissus, des vaisseaux sanguins ou des réseaux de vaisseaux sanguins humains ou animaux en utilisant un bioréacteur selon au moins une des exigences 1 à 11.

13. Procédé pour fabriquer un système de vaisseaux sanguins artificiel, alimentant, fabriqué au moyen de l'ingénierie tissulaire en utilisant un bioréacteur selon au moins une des exigences 1 à 11, le procédé comprenant au moins les étapes suivantes : le tissu reconstitué est introduit dans l'espace interne du bioréacteur et l'espace creux restant de l'espace interne est rempli avec le milieu nutritif liquide et perfusé par l'entrée et la sortie avec un milieu nutritif.

14. Utilisation d'un bioréacteur selon au moins une des exigences 1 à 11 pour la fabrication de tissus humains et animaux pour l'utilisation clinique et thérapeutique.

15. Utilisation d'un bioréacteur selon au moins une des exigences 1 à 11 pour le test de substances pharmacologiques, en particulier dans le domaine de la recherche cardiovasculaire et de l'obésité, la cicatrisation des plaies et l'oncologie.
